# EUROPÄISCHE PATENTSCHRIFT

(11) **EP 2 400 832 B1**
(45) Veröffentlichungstag und Bekanntmachung des Hinweises auf die Patenterteilung: **10.04.2019**
(21) Anmeldenummer: 10708797.5
(22) Anmeldetag: 24.02.2010
(51) Int. Cl.: A01G 7/00, A01G 25/16

(54) **SYSTEM UND VERFAHREN ZUR FERNÜBERWACHUNG VON TOPFPFLANZEN**
DEVICE AND METHOD FOR REMOTE CONTROL OF PLANTS IN POTS
DISPOSITIF ET PROCÉDÉ DE CONTRÔLE À DISTANCE DES PLANTES DANS DES POTS

(30) Priorität: 25.02.2009 DE 102009010579
(43) Veröffentlichungstag der Anmeldung: 04.01.2012
(73) Patentinhaber: Eidgenössische Technische Hochschule Zürich, 8006 Zürich (CH)
(72) Erfinder: KÖHLER, Moritz, CH-8001 Zürich (CH); BOLLIGER, Philipp, CH-8400 Winterthur (CH); OSTERMAIER, Benedikt, CH-8610 Uster (CH)
(74) Vertreter: Barth, Carl Otto
(86) Internationale Anmeldenummer: PCT/IB2010/000381
(87) Internationale Veröffentlichungsnummer: WO 2010/097689

(56) Entgegenhaltungen:
- WO-A2-02/35193
- WO-A2-02/084248
- GB-A- 2 341 007
- GB-A- 2 426 908
- US-A1- 2007 208 512

## Beschreibung

### Technisches Gebiet

Diese Erfindung betrifft die automatische Überwachung, Messung, Prüfung oder Untersuchung und entsprechende Anzeige bzw. Darstellung von ermittelten oder aus diesen bestimmten Werten, die für die Pflege und das "Wohlergehen" von Pflanzen von Bedeutung sind. Das dafür geeignete erfindungsgemässe System und das davon ausgeführte Verfahren erlauben es, dem Besitzer oder Benutzer ortsunabhängig den jeweiligen Zustand oder die Befindlichkeit einer Pflanze in einprägsamer, unmittelbar verständlicher Form mitzuteilen. Dabei wird dem Benutzer des Systems der aktuelle bzw. prognostizierte Zustand einer Pflanze in einer Weise übermittelt, die Emotionen der Pflanze simuliert und damit darauf abzielt, Emotionen beim Benutzer selbst auszulösen, z.B. durch sprach- und kulturunabhängige Icons. Weiterhin kann der Benutzer durch das erfindungsgemässe System benachrichtigt werden, sobald eine der überwachten Pflanzen Gefahr läuft, Schaden zu nehmen. Das System umfasst ein oder mehrere Pflanzgefäße, in die möglichst unauffällig Sensoren und zusätzliche Komponenten integriert sind, die vorzugsweise in Realzeit verschiedene Parameter ermitteln, z.B. Temperatur, Lichteinfall, Luft- und/oder Bodenfeuchtigkeit oder den Nähr- bzw. Schadstoffgehalt. Über ein lokales Netzwerk werden diese Parameter als digitalisierte Daten vorzugsweise drahtlos, z.B. über einen Gateway, einen Hub oder eine Verbindung zu einem Mobilfunknetz, an eine Zentrale bzw. einen zentralen Server geliefert. Dort findet der grösste Teil der Datenverarbeitung statt. Die dabei gewonnenen Zustandsgrössen für die jeweiligen Pflanzen werden vorzugsweise drahtlos an einen Empfänger, z.B. ein Mobilgerät, PDA, Handy oder PC übertragen und dem Benutzer in möglichst einfacher, aber einprägsamer Darstellung, z.B. durch sprach- und kulturunabhängige Icons, angezeigt.

### Hintergrund und Stand der Technik

Ein wesentliches Problem bei der Pflege von Pflanzen ist deren zeitverzögerte Reaktion auf äußere Einflüsse. Auch wenn erkannt wird, dass eine Pflanze nicht optimal versorgt ist, ist es oftmals schwierig, die konkrete Ursache zu bestimmen. Hinzu kommt, dass auch einmalige, kurzzeitige Ereignisse, wie z. B. ein vergessenes offenes Fenster im Winter, dauerhafte negative Auswirkungen auf eine Pflanze haben können.

Die aufgezeigten Probleme haben zu einer ganzen Reihe von Teillösungen geführt. Einige seien nachfolgend beschrieben.

In der veröffentlichten US Patentanmeldung 2007/0208511 ist die Architektur eines computerisierten Pflanzen-Überwachungsystems gezeigt, bei dem der Benutzer die Pflanze allerdings manuell klassifizieren muss. Ein Messfühler, der mit einer Speichereinrichtung versehen ist, wird für einige Zeit in den Boden gesteckt, danach herausgenommen und mit einem Computer verbunden, der die gemessenen und gespeicherten Daten ausliest. Ein entsprechendes Computerprogramm ermittelt dann den Zustand der Pflanze und zeigt diesen Zustand dem Benutzer an. Ein Nachteil dieses Systems besteht darin, dass keine Echtzeit-Beobachtung möglich ist, sondern nur die Werte der Vergangenheit ausgewertet werden können. Außerdem scheint es schwierig, das System für Pflanzen zu verwenden, die von unten bewässert werden. Weiterhin wird der Messfühler sichtbar sein, da er in die Erde gesteckt und zum Auslesen wieder entfernt werden muss.

Die veröffentlichte US Patentanmeldung 2007/0208512 zeigt ein Pflanzen-Überwachungsystem, das in Realzeit arbeitet und ebenfalls einen Messfühler und einem Computer aufweist. Der Messfühler enthält wiederum einen Speicher. Vom Computer wird in diesen Speicher vorab das Profil der Pflanze eingegeben, und der Messfühler in der Nähe der Pflanze platziert. Sobald nun in der Umgebung der Pflanze widrige Bedingungen festgestellt werden, die die Pflanze negativ beeinflussen können, zeigt dies der Messfühler an. Auch hier sind Messfühler und Computer tatsächlich nicht in Realzeit verbunden, sondern der Messfühler wird vorab mit dem Computer verbunden um die Profildaten der Pflanze zu speichern. Eine Änderung der Profildaten seit deren letzter Speicherung hat daher keine Auswirkung auf die Anzeige des Messfühlers.

In der veröffentlichten US Patentanmeldung 2007/0208591 ist ein System beschrieben, bei dem ein Computer benutzt wird, um Umgebungsdaten mit gespeicherten Pflanzenprofilen zu vergleichen. Damit soll festgestellt werden, welche Pflanzen für den gewählten Standort geeignet sind. Dabei wird dem Benutzer unmittelbar kommerzielle Information zum Erwerb solcher Pflanzen mitgeteilt.

In der veröffentlichten US Patentanmeldung 2007/0208592 ist ein ähnliches computerisiertes Auswahlsystem dargestellt, bei dem an dem Platz, an dem eine Pflanze angeordnet werden soll, eine Messeinrichtung installiert wird, die die Umgebungsdaten für eine gewisse Zeit aufnimmt und speichert. Die Messeinrichtung wird dann entfernt, die gemessenen Daten in einen Computer übertragen, ausgewertet und und es werden Vorschläge gemacht, welche Pflanzen für diesen Platz geeignet sind.

Im US Patent 7 400 975 ist ein Messfühler mit einem Speicher beschrieben, der in einer Architektur Verwendung finden kann, wie sie in der oben genannten US Patentanmeldung 2007/0208591 beschrieben ist. Der dargestellte Messfühler besteht aus zwei Teilen. Ein unterer Teil enthält eine Anzahl von Sensoren, die im Boden angeordnet sind. Der obere Teil ist ein abtrennbares Kommunikations-Interface, das die ermittelten Daten speichert. Das Kommunikations-Interface wird manuell vom Messfühler getrennt und mit einem Computer verbunden und kann so die gespeicherten Daten auf diesen übertragen. Auch hier handelt es sich nicht um eine Realzeit-Darstellung.

In der Veröffentlichung DE 10 2007 032 610 wird ein Verfahren zur Fernüberwachung des medizinischen Zustands eines Nutzers vorgeschlagen. Dabei erfolgt die Datenerfassung durch am Körper des Nutzers angebrachtes Sensoren, die über ein mehrstufiges Datenverarbeitungssystem zum Beispiel einen Arzt oder den Nutzer selbst benachrichtigen. In dieser Veröffentlichung sind weder Sensoren der in dieser Anmeldung genannten Art noch Messung von Temperatur, Feuchtigkeit, Schadstoffgehalt, etc. in einer Pflanzen-Umgebung vorgesehen.

In der Veröffentlichung EP 0 846 440 geht es um das Monitoring des physiologischen Status von Personen, insbesondere von Soldaten. Auch hier erfolgt die Datenerfassung durch am Körper des Soldaten angebrachte Sensoren und ein GPS. Ähnlich wie in vorheriger Veröffentlichung geht es hier nicht um eine Pflanzenumgebung, sondern das Messen in der Umgebung einer Person.

Auch in der Veröffentlichung EP 1 352 606 geht es um die Überwachung physiologischer Daten eines Patienten. Interessant ist hier, dass eine Reihe von Basisdaten vorab eingegeben werden und das Grenzwerte vorgesehen sind, bei deren Überschreitung eine Alarmmeldung erfolgt. Jedoch geht es auch hier nicht um die Überwachung von Pflanzen wodurch sich die vorliegende Anmeldung abgrenzt.

In der Offenlegungsschrift DE 10 2004 020 515 wird die Fernüberwachung von Muskelaktivitäten bei Menschen und Tieren beschrieben. Hierbei ist die eigentliche Messvorrichtung von der Datenverarbeitung getrennt. Am Patienten oder Tier wird ein kleines, batteriebetriebenes Gerät angebracht, dass die ermittelten Messwerte drahtlos überträgt. Die Abgrenzung zu dieser Veröffentlichung liegt wieder darin, dass in dieser keinerlei Bezug zu Pflanzen genommen wird.

Auch in der europäischen Patentanmeldung EP 1 002 496 geht es darum, Körperfunktionen eines Patienten kontinuierlich zu überwachen und auszuwerten. Diese Patentanmeldung befasst sich besonders mit der drahtlosen Fernüberwachung der ermittelten Messwerte, die mittels verschiedener Funk-Netzwerke durchgeführt wird. Weder die Kommunikation der Messwertgeber/Sensoren untereinander noch der Bezug auf eine Pflanzen-Umgebung sind offenbart.

In der Patentanmeldung US 2004/0 088 916 handelt es sich um ein Pflanzenüberwachungssystem mit einer Reihe von Sensoren, welche die ermittelten Daten per Funk übermitteln. Allerdings ist dieses System eher auf die Überwachung von kommerziellen Anbauflächen gerichtet und beschreibt keine Details wie sie in den Ansprüchen der vorliegenden Anmeldung enthalten sind. Hierdurch grenzt sich die vorliegende Anmeldung von der oben beschriebenen ab.

Diesen existierenden Teillösungen haften diverse Nachteile an. Ein Nachteil ist die Zeitverzögerung zwischen dem Erkennen widriger Umstände und der Benachrichtigung des Besitzers oder Benutzers, falls eine solche Benachrichtigung überhaupt vorgesehen ist. Ein anderer Nachteil ist die Beeinträchtigung des Aussehens der Pflanze, wenn z. B. Messfühler in der Nähe der Pflanze sichtbar sind. Auch ist es umständlich, wenn ein Messfühler zum Auslesen der Daten herausgenommen und in den meisten Fällen auch gesäubert werden muss, bevor er mit dem Computer verbunden werden kann. Außerdem erfordert Letzteres die Anwesenheit des Besitzers oder Benutzers. Den vorgestellten Systemen, die sich auf die Fernüberwachung von Personen beziehen, haftet demnach an, dass sie keinerlei Bezug auf die Überwachung von Pflanzen in Gebäuden oder in unmittelbarer Nähe eines Gebäudes nehmen. Die vorgestellten Systeme, die sich auf das Überwachen von Pflanze beziehen, sind entweder nicht für eine Fernüberwachung geeignet, oder ausschließlich für den Einsatz in kommerziellen Anbaugebieten geeignet. Im zweiten Fall liegt demnach keine Lösung vor, für die offensichtlich zu großen und störenden Sensoren in oder an einem Blumentopf.

Ein weiterer Nachteil der beschrieben Systeme liegt darin, dass sie jeweils nur Teillösungen darstellen. Mit anderen Worten, es findet keine Fusion der ermittelten Daten statt. Das Gesamtbild des Zustands einer Pflanze kann sich aber nur dann ergeben, wenn alle ermittelten Daten in die Darstellung dieses Bildes einfließen.

### Die Erfindung

Ausgehend von diesem Stand der Technik hat es sich die Erfindung zur Aufgabe gesetzt, ein integriertes System zu schaffen, das die Fernüberwachung einer Pflanze gestattet, wobei der Besitzer oder Benutzer in einfacher Weise frühzeitig und unmittelbar benachrichtigt werden soll, sobald widrige Umstände das Gedeihen bzw. die Gesundheit einer Pflanze in Frage stellen. Diese Benachrichtigung soll auch dann möglich sein, wenn sich der Besitzer oder Benutzer nicht in der Nähe des Objekts befindet. Auch extrapolierte Daten, d.h. Prognosen über zukünftige Sensorwerte (basierend u.a. auf historischen Werten, Pflanzenart, Standort, Jahreszeit, Wetterbericht, ...) können berücksichtigt werden. So kann sich die Pflanze in manchen Fällen rechtzeitig bemerkbar machen, falls sich ein Problem abzeichnet. Ein Beispiel für eine solche Benachrichtigung ist die Meldung "Brauche spätestens in 2 Tagen Wasser!", die den Benutzer informiert bevor ein Schaden entsteht. Auch soll der Besitzer keine besonderen Geräte auf sich tragen müssen, um die Benachrichtigung empfangen und sehen oder hören zu können. Die Erfindung kann auch so ausgestaltet sein, dass dem Besitzer oder Benutzer Anweisungen und Ratschläge gegeben werden können, wie er das Gedeihen und die Gesundheit des Objekts positiv beeinflussen kann. Ein wesentlicher Aspekt der Erfindung ist die Integration der technischen Komponenten in das Gefäss der Pflanze, um durch die Überwachung der Pflanze deren Aussehen und Ästhetik nicht zu beeinträchtigen. Außerdem soll das System einfach zu handhaben sein, d.h. ein Besitzer oder Benutzer muss mühelos damit umgehen können, ohne über besondere Computerkenntnisse verfügen zu müssen.

Um die beschriebene Funktionalität zu erreichen werden die jeweiligen Zustandsparameter der Pflanze in Realzeit unauffällig gemessen, übertragen, ausgewertet und eine Mitteilung oder ein entsprechendes Signal als Nachricht an den Besitzer oder Benutzer übermittelt.

Das erfindungsgemässe System und Verfahren zur Ermittlung und Anzeige von Zustand, Zustands- und Befindlichkeitsänderungen einer Pflanze, an einen Besitzer oder Benutzer zeichnet sich durch die Merkmale der Ansprüche 1 und 14 aus. Dabei sind Messfühler auf oder in einem Nährmedium ein einem Pflanzgefäß angeordnet, die lokal mindestens einen physikalischen Parameter ermitteln. Diese Messfühler sind zusammen mit anderen Komponenten in das Pflanzengefäß integriert, und ermöglichen die Kodierung des Parametersignals und dessen drahtlose Übertragung an einen Empfänger. Dieser Empfänger ist mit einem Prozessor verbunden, der die empfangenen Parametersignale verarbeitet, wobei das erzeugte Ausgangssignal eine Aussage über den Zustand des Objekts darstellt. Das erzeugte Ausgangssignal (z.B. Gesundheitszustand, Ort, Temperatur) wird dann drahtlos an ein vorzugsweise mobiles Anzeigegerät übertragen, welches dem Besitzer bzw. Benutzer eine optische oder akustische Anzeige über den Zustand der Pflanze vermittelt.

Dabei wird zumindest der Nähr- bzw. Schadstoffgehalt des Bodens bzw. der Nährlösung und vorzugsweise weitere der folgenden physikalischen Parameter ermittelt und ausgewertet:
- Temperatur und/oder Feuchtigkeit des Bodens,
- Temperatur und/oder Höhe der Wassersäule der Nährlösung
- Temperatur und/oder Feuchtigkeit der Umgebungsluft,
- Dauer und/oder Intensität und/oder Art (Polarisation, Lichtfarbe und -temperatur) des auf die Pflanze fallenden Lichts.

Wie in der Beschreibung der Erfindung im nächsten Abschnitt beschrieben, stellt ein intelligenter Blumentopf einen wesentlichen Teil der Erfindung dar. Unter einem "intelligenten Blumentopf" soll hier ein Blumentopf verstanden werden, der durch Sensoren erweitert wurde, die einen oder mehrere der oben genannten physikalischen Parameter messen können. Überdies verfügt ein intelligenter Blumentopf über einen Prozessor und Speicher um die Signale der Sensoren verarbeiten zu können. Dieser intelligente Blumentopf kann die Sensordaten über ein drahtloses Kommunikationsmodul wahlweise direkt oder mithilfe einer Relaisstation ins Internet übertragen, wo eine Weiterverarbeitung der Daten stattfindet. Einzelheiten des erfindungsgemässen Systems und Verfahrens können der nachfolgenden Beschreibung und den Patentansprüchen entnommen werden.

### Beschreibung mehrerer Ausführungsbeispiele

Nachfolgend wird die Erfindung an Hand einiger Ausführungsbeispiele, die auch in den dazugehörigen Zeichnungen dargestellt sind, im Detail erläutert. Auf den Zeichnungen zeigen:
- Fig. 1: eine Übersicht über das gesamte System;
- Fign. 2a-2e: eine Teilausführung des intelligenten Blumentopfs;
- Fig. 3: ein Beispiel für einen unauffälligen sog. Sensorclip;
- Fign. 4a-4b: Beispiele für Sensorclip-Anordnungen;
- Fign. 5a-5e: Beispiele für intelligente Blumentöpfe; und
- Fign. 6a-6b: Beispiele für eine Ausführung eines intelligenten Blumentopfes bestehend aus Sensoren, die in das Substrat gesteckt werden.

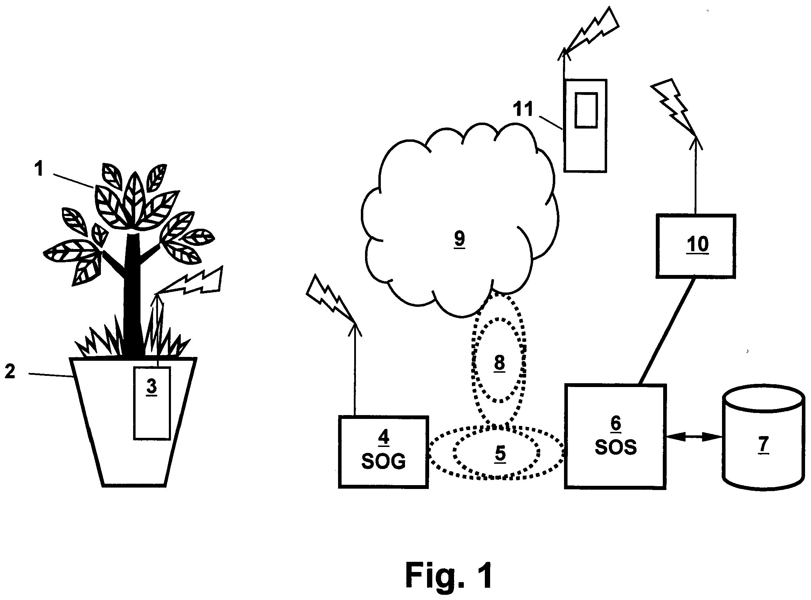

In Fig. 1 ist beispielhaft eine Übersicht über das gesamte System dargestellt. Eine Pflanze 1 befindet sich in einem Blumentopf 2, der mit Substrat bzw. Erde, Blähton oder mit einem Hors-Sol-Material wie Kokosfasern oder Steinwolle gefüllt ist. In der nachfolgenden Beschreibung und in den Patentansprüchen soll unter "Erde" jedes Material verstanden werden, in dem Pflanzen wurzeln oder wachsen können, also z.B. auch Blähton oder ein Hors-Sol-Material.

Ebenfalls im Blumentopf ist ein Sensor 3 gezeigt, der beispielsweise den Wasserstand misst. Wie weiter unten beschrieben, wird nicht nur ein einzelner Sensor oder Sensorknoten verwendet, sondern es werden mindestens zwei Sensoren für die verschiedensten Parameter-Messungen im oder am Blumentopfes angeordnet sein.

Natürlich könnten die Parameter-Signale direkt vom Blumentopf 2 ins Internet 5 übertragen werden. Dazu benötigte der Blumentopf 2 eine Sende-Empfangseinheit, die standardkonform zu einer bereits existierenden Infrastruktur ist, wobei hier z.B. GSM, UMTS oder IEEE 802.11 in Frage kämen. Diese Standards sind jedoch für hohe Datenübertragungsraten (UMTS, IEEE 802.11) oder mobile Datenübertragung (UMTS, GSM) entwickelt worden. Der Preis für diese Funktionalität ist in der Regel ein sehr höherer Energieverbrauch des Senders. Im vorliegenden Fall werden jedoch nur geringe Datenmengen übertragen und die Endgeräte sind stationär. Daher können hier energetisch sparsame Standards angewandt werden, z.B. Zigbee, Z-wave oder 6LoWPAN/802.15.4. Für diese Standards gibt es allerdings keine ubiquitäre Infrastruktur, d.h. es muss vor Ort eine Infrastruktur geschaffen werden. Genau dazu dient der optionale Smart Object Gateway (SOG) 4. Eine Übertragung über eine vorhandene aber energieaufwendige Infrastruktur (z.B. GSM, UMTS, oder IEEE 802.11) kann dennoch Sinn machen, wenn die Anzahl der Datenübertragungsvorgänge gering ist. Beispielsweise macht es durchaus Sinn auf einen energieaufwändigen Standard zurückzugreifen, wenn nur ein Mal täglich Daten übermittelt werden.

Jedoch ist anzumerken, dass der SOG 4 sehr wohl auf eine bereits vorhandene drahtlose Infrastruktur (z.B. GSM, UMTS, IEEE 802.11 oder 802.3) zurückgreifen kann um die Daten ins Internet 5 zu übermitteln. Insbesondere kann der SOG natürlich z.B. auch ein GSM, UMTS, oder IEEE 802.11 Zugangspunkt sein, d.h. der SOG kann Teil einer unabhängigen Telekommunikationsinfrastruktur sein. Er fungiert somit als Gateway zwischen einer energetisch günstigen und einer energetisch weniger günstigen Übertragungstechnologie.

Es werden also die von dem (oder den) intelligenten Blumentöpfen 2 abgegebenen digitalen Ausgangssignale, die den (oder die) genannten Parameter darstellen, wahlweise über den beschriebenen Gateway SOG 4 an einen zentralen Computer bzw. Server 6 übertragen.

Dieser zentrale Computer bzw. Server wird hier als Smart Object Server (SOS) 6 bezeichnet. Dort findet eine zentrale Datenverarbeitung statt, wobei auf existierende, in einem Speicher 7 befindliche Daten zugegriffen werden kann. Weiterhin kann der SOS 6 auch auf externe Datenquellen 9 zugreifen, was über die Internet-Verbindung 8 erfolgt. Damit wird es möglich, den genauen Status der beobachteten Pflanze 1 zu bestimmen, Fehlentwicklungen und Möglichkeiten zu deren Behebung zu erkennen, mögliche Gefahren abzuschätzen, kurz gesagt, ein umfassendes Gesamtbild des beobachteten Objekts 1 zu erstellen.

Der Aufbau bestehend aus einem intelligenten Blumentopf, der drahtlos Daten entweder direkt oder über einen Smart Object Gateway an einen Smart Object Server übermittelt, wird als bekanntes Standardverfahren angenommen. Der intelligente Blumentopf ist in diese übergeordnete Architektur eingebettet.

Das Gesamtbild der überwachten Pflanze wird nun dem Besitzer oder Benutzer der Pflanze übermittelt. Dies kann nun ebenfalls über das Internet erfolgen, mit dem der SOS 6 bereits verbunden ist. (Diese Möglichkeit ist in Fig. 1 nicht dargestellt.) Eine elegantere Methode besteht darin, ein mobiles Endgerät, welches über eine drahtlose Netzwerkschnittstelle verfügt (wie z.B. ein Mobiltelefon bzw. Smartphone) als Benutzerschnittstelle zu verwenden und dem Besitzer in augenfälliger Weise den Zustand seiner Pflanze (oder einer Vielzahl seiner Pflanzen) anzuzeigen. Dabei kann sowohl eine automatische Avisierung des Benutzers oder Besitzers vorgesehen sein, z.B. bei Wassermangel der Pflanze und damit der Gefahr des Verdorrens, oder auch eine vom Besitzer gestartete Anfrage an das System nach dem Zustand oder Befinden der Pflanze. Die Darstellung des jeweiligen Zustands oder der Befindlichkeit einer Pflanze wird in einprägsamer, unmittelbar verständlicher Form durchgeführt. Dazu wird dem Benutzer des Systems der aktuelle bzw. prognostizierte Zustand der Pflanze in einer Weise übermittelt, die Emotionen der Pflanze simuliert und damit darauf abzielt, Emotionen beim Benutzer selbst auszulösen, z.B. durch sprach- und kulturunabhängige Icons.

Dies geschieht beides über ein Telekommunikationsnetz, welches vorzugsweise das Internet-Protokoll unterstützt, insbesondere das Mobilfunknetz, z.B. nach dem GSM- oder UMTS-Standard. Dazu ist der SOS 6 mit einem oder auch mehreren Mobilfunkbetreiber(n) 10 verbunden, von dem oder von denen aus die Verbindung über das Mobilfunknetz mit dem Mobilfunkgerät 11 des Benutzers oder Besitzers der Pflanze 1 hergestellt wird. Die inhaltliche Steuerung dieser Kommunikation erfolgt in erster Linie durch den SOS 6.

Die Funktion des erfindungsgemässen Systems sei an folgenden Beispielen erläutert.

### Beispiel 1:

Der Benutzer oder Besitzer des Systems möchte sich, während er sich in den Ferien befindet, über die Vitalität einer seiner Pflanzen informieren. Dazu startet er eine Applikation auf seinem Mobiltelefon 11, welche sich mit dem SOS 6 verbindet. Da der smarte Blumentopf, in dem sich die Pflanze befindet, über die gesamte Zeit Messwerte an den SOS 6 schickt, ist es nun möglich, dort aufgrund von aktuellen und historischen Daten sowie durch den Vergleich von anderen, gleichartigen Pflanzen einen detaillierten Zustandsbericht zu berechnen. Dieser Zustand wird nun auf das Mobiltelefon des Benutzers 11 geschickt und z.B., da es der Pflanze gut geht, mit einem lachenden Smiley angezeigt.

### Beispiel 2:

Nach der Installation gibt es auf dem SOS 6 eine logische Verknüpfung zwischen einem Benutzer, einem Blumentopf und der Pflanze, die sich im entsprechenden Topf befindet. In regelmäßigen Abständen oder beim Eintreten eines außerordentlichen Ereignisses übermittelt der Blumentopf Sensorwerte an den SOS 6. Dies lässt sich gut am Beispiel Wassermangel erklären. Da sich der Wasserstand in der Pflanze nur äußerst langsam ändert, reicht es aus, den Wasserstand z.B. einmal täglich zu übermitteln. Natürlich kann der Wasserstand bei Bedarf auch mit einer höheren oder niedrigeren Abtastrate gemessen werden. Der Benutzer ist jederzeit in der Lage, den aktuellen Wasserstand abzufragen, da die Kommunikation zwischen seinem Mobiltelefon und dem SOS 6 stattfindet. Fällt der Wasserstand unter eine Toleranzmarke, wird ein Alarm erzeugt, und der Benutzer erhält eine Nachricht auf seinem Mobiltelefon. Wenn der Benutzer nun die entsprechende Pflanze giesst, visualisiert das System den Wasserstand auf dem Mobiltelefon in Echtzeit. Dies geschieht dadurch, dass ein steigender Wasserstand als außerordentliches Ereignis interpretiert wird, da schließlich nur in einem Bruchteil der gesamten Systemzeit ein Gießvorgang vorgenommen wird. Dadurch werden die Daten nicht in regelmäßigen Abständen, sondern nur beim Verlassen eines Toleranzbereiches übermittelt. Im Fall des Wasserstands wäre dieser Bereich also sehr klein, was zur Folge hat, dass auch nur geringe Veränderungen als außerordentliches Ereignis interpretiert werden. Dieses Ereignis wiederum löst aus, dass durch die Nachrichtenkette Sensorknoten, SOG, SOS, und schließlich Mobiltelefon die Nachrichten so schnell übermittelt werden können, dass dem Benutzer ein Nachverfolgen der eigenen Aktivität, also des Giessens, in Echtzeit ermöglicht wird.

Wie vorgehend bereits erläutert, liegt der Kern der Erfindung in der möglichst unauffälligen Messung von Parametern durch Sensoren und deren drahtlosen Übermittlung. Diese Angabe wird dem Benutzer oder Besitzer des Objekts praktisch in Realzeit übermittelt. Im dargestellten System werden folgende Parameter gemessen:
- Temperatur des Bodens
- Feuchtigkeit des Bodens bzw. Wasserstand im Reservoir
- Nähr- bzw. Schadstoffgehalt des Bodens bzw. des Wassers im Reservoir
- Temperatur der Umgebungsluft
- Feuchtigkeit der Umgebungsluft
- Lichtstrom und/oder Beleuchtungsstärke des auf das Objekt fallenden Lichts.

Insgesamt gesehen werden die oben beschriebenen Aufgaben durch die Integration von Sensoren und diverse Einrichtungen zur Datenübertragung, Datenverarbeitung und Datenspeicherung gelöst. Da es unterschiedliche Arten von Pflanzen gibt, besteht die Notwendigkeit, unterschiedliche Lösungsansätze zu benutzen. Dies sei am Beispiel einer Wasserstandsmessung nachfolgend gezeigt.

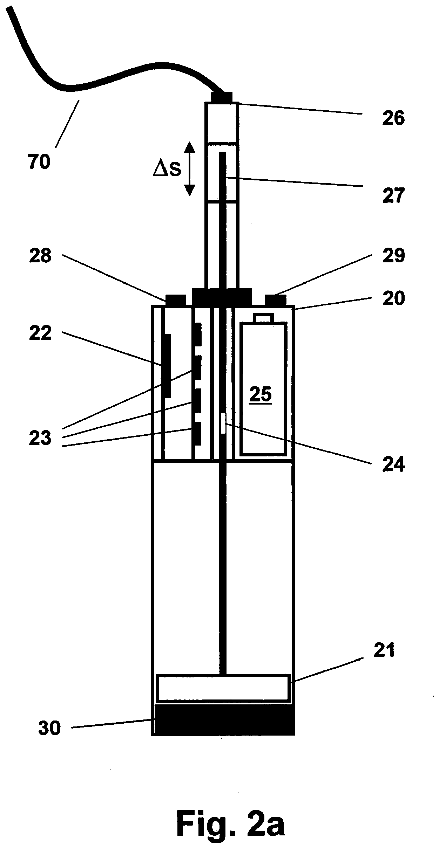

Das vorliegende technische Problem besteht darin, zuverlässig und möglichst unsichtbar den Wasserstand im Topf einer Topfpflanze zu ermitteln. Dies hängt vom Bewässerungssystem des Topfes ab. Bei einem Unterflur-System (sub-irrigation system) wird die Pflanze mit Wasser von unten versorgt. Im Topf befinden sich üblicherweise Erde und/oder Tonkugeln (Blähton), wobei letztere das Wasser aufnehmen. Ein dafür geeigneter Sensor ist in Fign. 2a-2d gezeigt und nachfolgend beschrieben.

Die Figuren 2a bis 2d zeigen Beispiele für Sensoren, wie sie in dem erfindungsgemässen System Verwendung finden können und mit deren Hilfe die Wassersäule in einem Unterflurbewässerungssystem gemessen werden kann. Diese Sensorknoten messen den Wasserstand und verschiedene andere physikalische Grössen und erzeugen digitale Ausgangssignale, die sie per Funk übertragen.

Der in Fig. 2a dargestellte Sensor besteht aus einem Gehäuse 20, in dem ein in vertikaler Richtung beweglicher Schwimmer 21 angeordnet ist, der an einer fest mit ihm verbunden Stange einen Magneten 24 trägt. An der dem Magneten 24 gegenüberliegenden Wand ist eine Reihe von Reed-Schaltern 23 - im gezeigten Beispiel vier - angeordnet, so dass bei verschiedenen Stellungen des Schwimmers 21 unterschiedliche Reed-Schalter aktiviert werden. Die Stellung des Schwimmers 21 ist vom Wasserstand abhängig, so dass die vom Magneten 24 geschlossenen Reed-Schalter unmittelbar eine digitale Erfassung des Wasserstands abgeben. Es reicht z.B. aus, einen Reed-Schalter je cm Wasserstandhöhe vorzusehen, wobei dies auch von Länge und Feldstärke des verwendeten Magneten 24 abhängt. Die Anzahl der Reed-Schalter hängt natürlich auch von der Differenz zwischen minimalem und maximalem Wasserstand ab. Einzelheiten der Digitalisierung des Wasserstands sind nachfolgend beschrieben.

Weiterhin befinden sich an oder in dem in Fig. 2a dargestellten Sensor noch eine Batterie 25 für die Stromversorgung und ein elektronischer Schaltkreis 22, der zur Verarbeitung bzw. Digitalisierung des oder der ermittelten Parameter-Werte und zur Erstellung eines per Funk übermittelbaren Datensatzes dient. Dieser wird über die Antenne 27 gesendet.

Folgende Sensoren können beispielsweise zusätzlich im oder am Wasserstandsanzeiger angeordnet sein: ein Lichtsensor 26, ein Luftfeuchtigkeits-Sensor 28, ein Temperatursensor 29, wobei diese drei vorzugsweise im oberen Bereich des Wasserstandsanzeigers angeordnet sind. Am Boden oder im unteren Bereich ist weiterhin ein Nährstoff- bzw. Schadstoffsensor 30 angebracht, der die Konzentration der sich im Topf befindenden Nähr- bzw. Schadstoffe erfasst. Um Verschattung vorzubeugen kann der Lichtsensor optional um einen Lichtwellenleiter 70 erweitert werden. Dieser kann zum oberen Bereich der Pflanze geleitet werden um dort einen Teil des Mikroklimas zu messen.

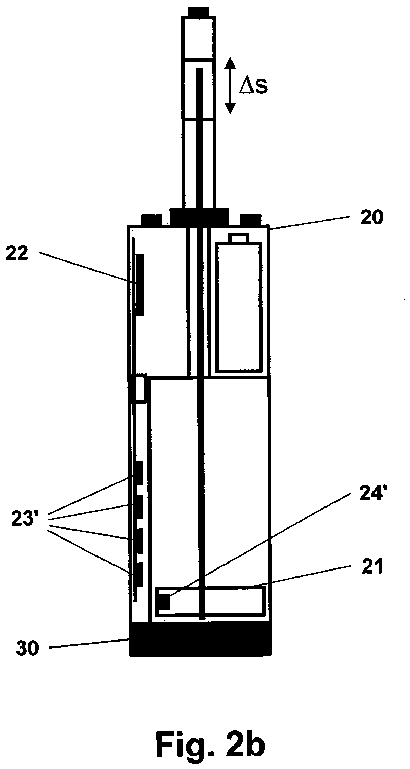

Der in Fig. 2b dargestellte Wasserstandsanzeiger besteht im Prinzip aus den gleichen Teilen wie der Wasserstandsanzeiger in Fig. 2a, die Einzelteile sind daher hier nicht nochmals aufgeführt. Im Gehäuse 20 ist wiederum der Schwimmer 21 angeordnet ist, wobei sich hier der Magnet 24' im oder am Schwimmer 21 befindet. An der dem Magneten 24' gegenüberliegenden Wand befinden sich vier Reed-Schalter 23', so dass bei verschiedenen Stellungen des Schwimmers 21 unterschiedliche Reed-Schalter betätigt werden.

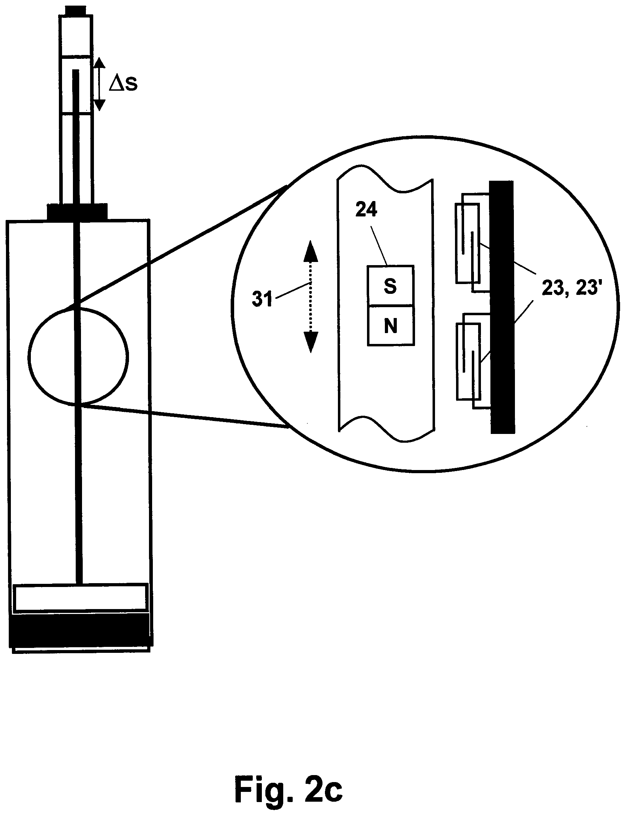

In Fig. 2c ist die Anordnung von Magnet 24 und Reed-Schalter 23 nochmals in einer vergrößerten Detailansicht gezeigt, wobei diese Figur wohl keiner weiteren Erläuterung bedarf.

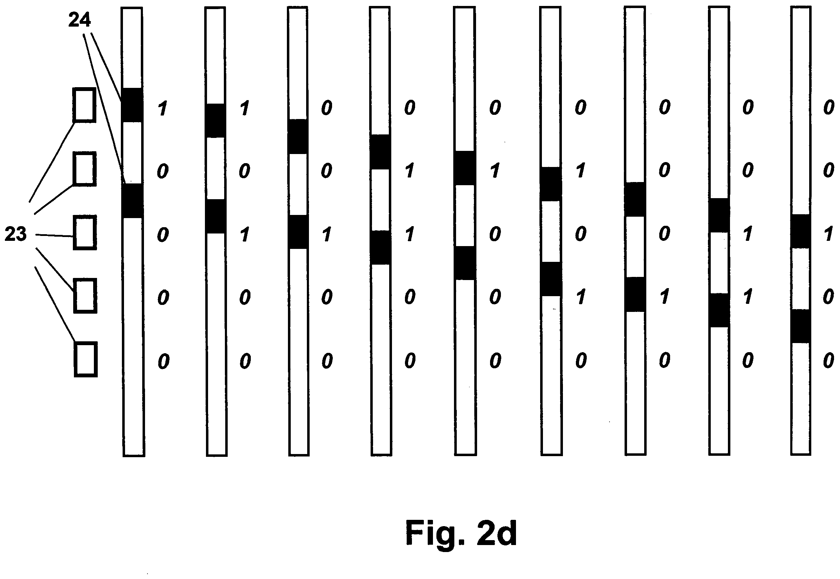

Natürlich kann mehr als ein Magnet 24 mit dem Schwimmer 21 verbunden werden; damit lässt sich z. B. die Auflösung der Anordnung verbessern. In Fig. 2d sind insgesamt fünf Reed-Kontakte 23 gezeigt, die einem Schwimmer mit zwei Magneten 24 gegenüber stehen.

Diese Fig. 2d zeigt, wie das digitalisierte Ausgangssignal des Wasserstandes unmittelbar aus den Stellungen des Schwimmers entsteht. In diesem Fall wird unmittelbar ein digitales Signal erzeugt, das den Wasserstand anzeigt. Im gezeigten Beispiel sind zwei Magnete 24 am Schwimmer angebracht oder fest mit ihm verbunden, dazu fünf Reed-Schalter 23 gegenüber den Magneten; dabei sind die Abstände der Reed-Schalter untereinander geringer als die Abstände der beiden Magneten.

In Fig. 2d sind verschiedene Stellungen der Magnete 24 von links nach rechts dargestellt, die verschiedenen Wasserständen entsprechen. In der ersten Darstellung oder Spalte ganz links befindet sich der obere Magnet gegenüber dem obersten Reed-Schalter, so dass dieser schliesst. In einer fünfstelligen, binären Digitalzahl entspricht dies einer ersten "1" mit vier folgenden "0"; die vollständige Digitalzahl für den oberen Grenzwasserstand lautet demnach "1 0 0 0 0". Sinkt der Wasserstand, z.B. durch Verbrauch und Verdunstung, so bewegt sich der Schwimmer mit den Magneten 24 etwas nach unten und nimmt die in der zweiten Spalte dargestellte Stellung ein. Der oberste Reed-Schalter bleibt geschlossen, dazu ist der dritte Reed-Schalter von oben ebenfalls geschlossen: die entsprechend ausgegebene Digitalzahl lautet "1 0 1 0 0". Bei weiterem Absinken des Wasserstands stehen die in den nächsten nach rechts verlaufenden Spalten dargestellten Digitalzahlen "0 0 1 0 0", "0 1 1 0 0", etc. als Anzeige des jeweiligen Wasserstands zur Verfügung. Die letzte Spalte zeigt den unteren Grenzwasserstand; die zugehörige Digitalzahl lautet "0 0 1 0 0".

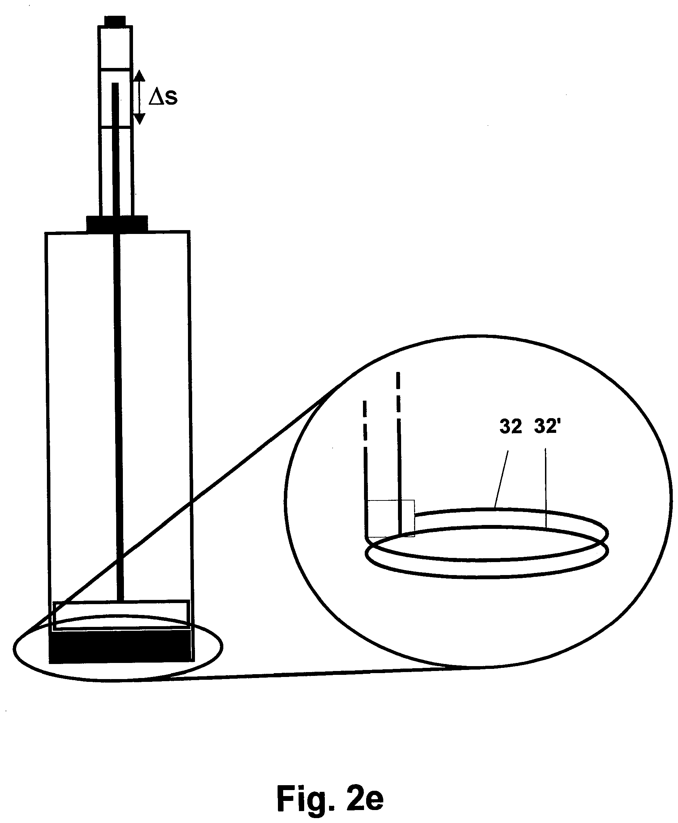

Figur 2e zeigt den Nähr- bzw. Schadstoffsensor 30 im Detail. Zur Nährstoffmessung wird beispielsweise die Leitfähigkeit des Wassers gemessen, welche ein Maß für die Salinität, d.h. den Salzgehalt, des Wassers darstellt. Dazu werden die zwei Messelektroden 32, 32' ringförmig am Innenrand, d.h. auf der Innenseite des Gehäuses 20 angebracht. Um den Schwimmer 21 nicht am Auf- bzw. Absteigen zu hindern, werden die Messelektroden 32, 32' so im Gehäuse integriert, dass sich die Oberfläche des Gehäuses 20 nicht verändert. Als Elektrodenmaterial kann ein korrosionsarmes Metall oder ein anderes leitfähiges Material verwendet werden. Hierzu kommen insbesondere Grafit, Platin und Titan in Frage, wobei es aus Kostengründen vorteilhaft ist, Titan bzw. eine Titanlegierung zu verwenden. Insbesondere ist Titan vom Grad 2 hierzu geeignet. Zur Messung wird für kurze Zeit ein Strom an die beiden Messelektroden 32, 32' angelegt und mittels des elektronischen Schaltkreises 22 der elektrische Widerstand zwischen den Messelektroden 32, 32' bestimmt. Der Kehrwert des Widerstandes wird als Leitfähigkeit des Wassers bezeichnet und dient zur Bestimmung von dessen Salinität. Auf diese Weise kann man die wichtigsten anorganischen Nährstoffverbindungen nachweisen, zu denen insbesondere Stickstoff und stickstoffhaltige Verbindungen, Kalium und Phosphor zählen.

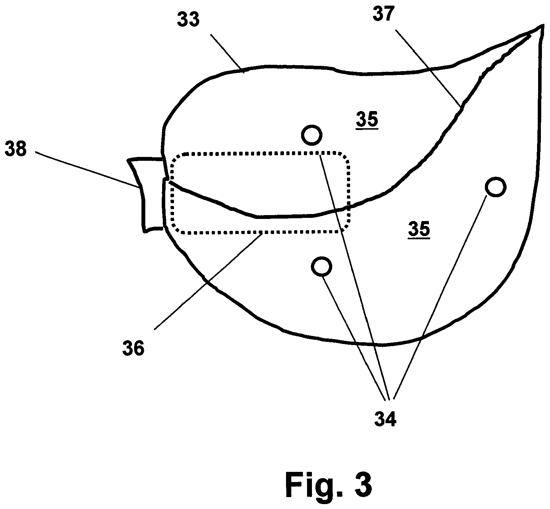

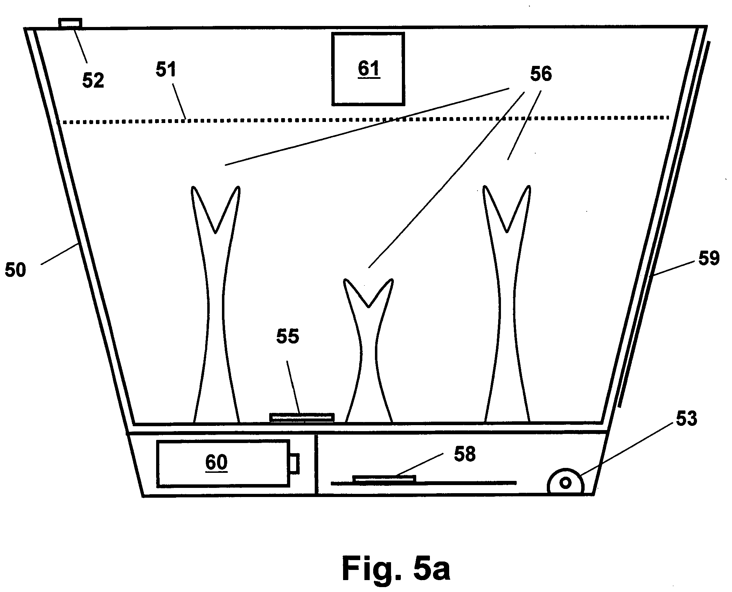

Weiterhin kann der intelligente Blumentopf als Relaisstation für die Weiterleitung von Daten oder Datenpaketen benutzt werden, die er von anderen Sensoren erhält, insbesondere solchen mit geringer Sendeleistung, wie z.B. den sog. "Sensorclips". Dies ist deshalb möglich, weil der Blumentopf gemäss den Figuren 5a bis 5d im Vergleich zum "Sensorclip" aus Fig. 3 eine relativ leistungsfähige Stromversorgung enthält. Ein entsprechendes Beispiel eines "Sensorclips" mit schwacher Stromversorgung wird nachfolgend beschrieben.

In Fig. 3 ist ein anderer, nämlich in Form eines Blattes, einer Frucht oder einer Blüte gestalteter Sensorknoten gezeigt, der nachfolgend als "Sensorclip" bezeichnet wird. Auch dieser Sensorclip enthält mehrere Sensoren, die geeignet sind, z.B. Temperatur und Feuchtigkeit derjenigen Luft zu messen, welche die Pflanze umgibt, d. h. deren Mikroklima. Ein weiterhin auf dem Sensorclip angeordneter, lichtempfindlicher Sensor dient dazu, das einfallende Licht zu messen, womit auch dessen zeitliche Verteilung bestimmt werden kann.

Der Sensorclip in der Fig. 3 ist eine insofern vereinfachte Variante eines Sensorknotens, als er auf einen besonders niedrigen Stromverbrauch ausgelegt ist. Damit wird es möglich, ihn mittels einer Solarzelle zu betreiben, womit eine Batterie entfallen kann oder ggf. nur als kleine Pufferbatterie notwendig ist. Der erwünschte niedrige Stromverbrauch resultiert allerdings in einer geringen Reichweite beim Senden der ermittelten Daten. Auch die Weiterleitung von empfangenen Datenpaketen scheidet damit aus, wie sie von anderen Sensorknoten durchgeführt werden kann.

Der Sensorclip sollte ausserdem ein geringes Gewicht aufweisen, damit er in einfacher Weise an oder in der Nähe der Pflanze, z.B. unauffällig an deren Stamm, angeordnet werden kann, ohne den Gesamteindruck zu beeinflussen.

Der in Fig. 3 schematisch dargestellte Sensorclip 3D3 in Form eines Blattes besteht aus einem Träger 35, beispielsweise einer Kunststofffolie, die gleichzeitig als Solarzelle ausgebildet sein kann. Am "Stiel" befindet sich ein Befestigungsteil 38, mit dem der Sensorclip 33 an der Pflanze selbst oder an einem Halter angebracht werden kann. Die Solarzelle auf der Oberfläche des Sensorclips dient zur Stromversorgung. Ebenfalls auf der Oberfläche sind bei dem dargestellten Sensorclip insgesamt drei Sensoren 34 angeordnet. Diese messen das einfallende Licht, die herrschende Temperatur und die Feuchtigkeit der Umgebungsluft. Die Sensoren und die Solarzelle sind mit einem elektronischen Schaltkreis 36 verbunden, der sich praktisch unsichtbar auf der Unterseite des Blattes befindet. Dieser elektronische Schaltkreis 36 übernimmt die Auswertung der von den Sensoren ermittelten Werte und die Übermittlung der daraus gewonnenen digitalen Parametersignale mittels eines Senders über die Antenne 37 an den Gateway 4 oder eben im Falle einer Relaisstation zunächst an den intelligenten Blumentopf, wobei hier in der Regel nur geringe Distanzen von 1-2m überwunden werden müssen.

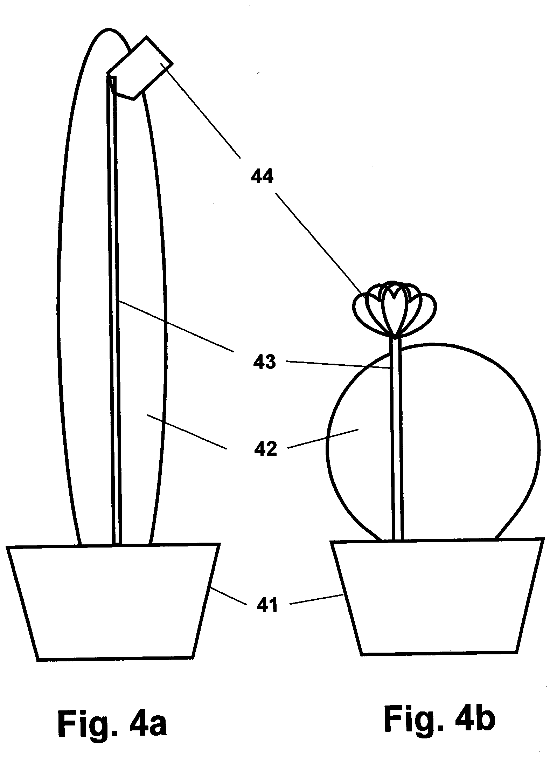

Geeignete Anordnungen eines solchen Sensorclips sind in den Figuren 4a und 4b gezeigt. In beiden Fällen ist der Sensorclip 44 an einer Stange 43 befestigt, die neben der Pflanze 42 in der Erde des Blumentopfes 41 steckt. In Fig. 4a ist der Sensorclip 44 als Blatt ausgebildet, wie in den Fig. 3 gezeigt. In Fig. 4b ist der Sensorclip 44 als Blüte ausgebildet und auch damit relativ unauffällig neben der Pflanze.

Es gibt auch Pflanzen, deren Blätter regelmäßig befeuchtet werden müssen. An einer solchen Pflanze kann ein auf einem Blatt angebrachter Sensorclip dazu dienen, den Vorgang des Besprühens festzustellen oder sogar einen Hinweis zu geben, dass die Pflanze mit Wasser besprüht werden muss.

Bei einen Oberflächen-Bewässerungssystem, bei dem die Wasserversorgung von oben her erfolgt, kann ein Sensor gemäss den Figuren 2a oder 2b nicht verwendet werden. In einem solchen System ist üblicherweise der gesamte Topf mit Erde gefüllt, womit die Boden-Feuchtigkeit nicht über den Wasserstand ermittelbar ist. Hinzu kommt, dass ein von oben in die Erde eingeführter Sensor zu ungenau misst, da sich einerseits das Wasser im unteren Bereich sammelt und dort sogar zu Fäulnis führen kann, andererseits die Erde von oben nach unten durchtrocknet. Dazu gibt es mehrere Lösungsansätze.

Man kann, wie es im Stand der Technik gelöst ist, einen von oben in die Erde gesteckten Feuchtigkeitssensor benutzen, um die Feuchtigkeit der Erde zu messen. Da jedoch die Erde von oben nach unten durchtrocknet, ist die Feuchtigkeit an der Oberfläche nur bedingt aussagekräftig und damit eine solche Messung sehr ungenau.

Ein völlig anderer Ansatz besteht darin, die Feuchtigkeit erfindungsgemäss in der Erde im Blumentopf an mehreren Stellen, ggf. auch in verschiedenen Höhen zu messen, und im oder am Topf noch weitere Sensoren zur Bestimmung weiterer Parameter anzubringen. M.a.W., es wird kein Sensor an oder in einem gewöhnlichen Blumentopf angeordnet, sondern der Topf selbst wird innen und/oder aussen mit mehreren Sensoren ausgerüstet, die entsprechende Daten liefern. Ein solcher "intelligenter Blumentopf" ist in den Figuren 5a bis 5d in verschiedenen Ausführungen gezeigt und wird nachfolgend genauer beschrieben.

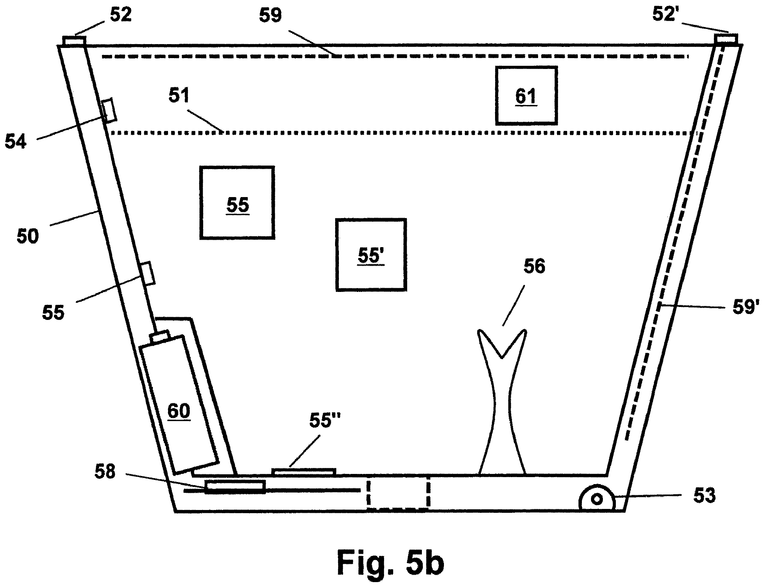

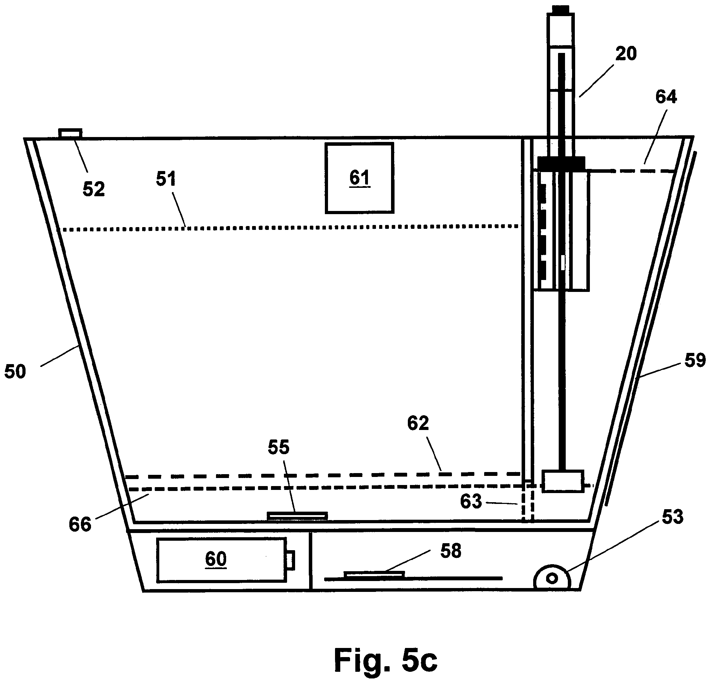

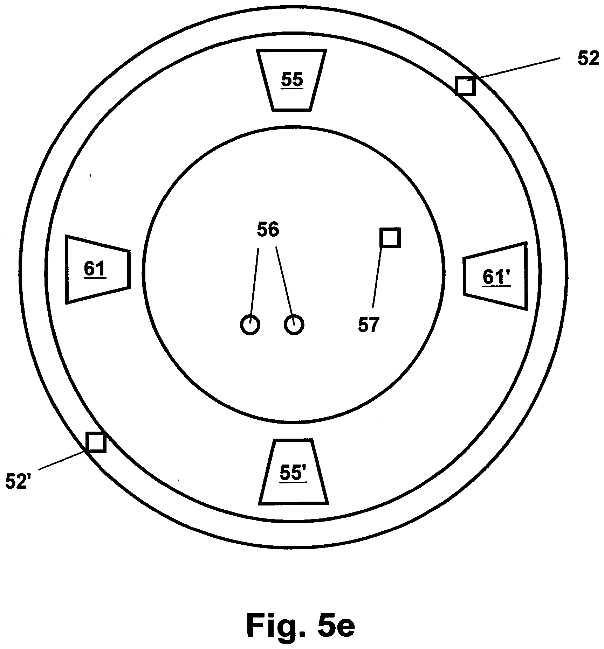

Fig. 5a zeigt ein erstes Ausführungsbeispiel eines solchen intelligenten Blumentopfes im Querschnitt, welches einem Oberflächenbewässerungssystem entspricht; Fign. 5b und 5c ein zweites, modifiziertes Ausführungsbeispiel, welches einem Unterflurbewässerungssystem entspricht; Fig. 5e schliesslich eine Draufsicht.

Der Topf 50 in Fig. 5a ist bis zum Niveau 51 mit Erde, Blähton oder einem Hors-Sol-Material gefüllt. Auf der oberen Kante, dem Rand des Topfes 50, ist mindestens ein lichtempfindlicher Sensor, Lichtsensor 52, angeordnet. Es können auch mehrere solche Lichtsensoren über den Rand des Topfes 50 verteilt sein. Weiterhin befinden sich im Topf eine Anzahl stabförmiger Elektroden 56, die vom Boden des Topfes ausgehend, sich in die Erde erstrecken. Diese dienen als Erdfeuchtigkeits-Sensor. Je mehr Elektroden im Topf angeordnet werden, desto genauer ist die Messung, da zwischen verschiedenen Bereichen des Topfes unterschieden werden kann. Die Elektroden sind in Fig. 5a ungleich lang dargestellt, können aber auch gleiche Länge aufweisen. Der Vorteil dieser Anordnung ist, dass die Erdfeuchtigkeit tatsächlich in der Nähe der Wurzeln der Pflanze und nicht an der häufig ausgetrockneten Erdoberfläche bestimmt wird. Die Elektroden selbst messen elektrische Kapazität, die ein Mass ist für den den sog. dielektrischen Wert, welcher ein Grad für die relative Permittivität ist. Diese kann insbesondere durch eine Frequenzmessung bestimmt werden.

Weiterhin ist am Boden des Topfes 50 ein Temperatursensor 53 für die Messung der dort herrschenden Temperatur vorgesehen.

Ebenfalls am Boden des Topfes 50 innerhalb der Erde befindet sich ein Nährstoffsensor 55, der den Nährstoff- oder Schadstoffgehalt der Erde bestimmt.

Ein elektronischer Schaltkreis 58 ist isoliert und abgedichtet in den Boden des Topfes 50 eingelassen. Er dient zur Verarbeitung, insbesondere Digitalisierung der von den Sensoren ermittelten Daten und bereitet die Datenpakete auf, die für die drahtlose Übermittlung vorgesehen sind. Letztere erfolgt über die Antenne 59, die sich an der Aussenwand des Topfes 50 befindet.

Die Stromversorgung erfolgt durch eine Batterie 60, die ebenfalls isoliert und abgedichtet in den Boden des Topfes 50 eingelassen ist, allerdings so, dass sie ohne Entleerung des Topfes gewechselt werden kann. Eine Versorgung durch eine externe Stromquelle, insbesondere einer Steckdose ist auch möglich.

Der modifizierte Topf 50 in Fig. 5b ist ebenfalls bis zum Niveau 51 mit Erde gefüllt. Auf der oberen Kante, dem Rand des Topfes 50 sind zwei lichtempfindlicher Sensoren, Lichtsensoren 52 und 52', angeordnet. Natürlich können auch hier mehrere solche Lichtsensoren über den Rand des Topfes 50 verteilt sein.

Oberhalb des Niveaus 51 der Erde im Topf 50 ist weiterhin ein Feuchtigkeitssensor 54 angeordnet, der die Luftfeuchtigkeit im Topf nahe der Erdoberfläche ermittelt.

Im oberen Bereich des Topfes 50, jedoch unterhalb des Niveaus 51 befinden sich zwei Nährstoffsensoren 55 und 55' die den Nährstoffgehalt der Erde im Topf überwachen. Ein dritter Nähstoffsensor 55", der z.B. als Stickstoffsensor ausgebildet sein kann, befindet sich am Boden des Topfes innerhalb der Erde.

Ausserdem sind am Boden des Topfes 50 wiederum zwei stabförmige Elektroden angeordnet, die als Erdfeuchtigkeitssensor 56 fungieren. Sie ragen in die Erde hinein und bestimmen die Feuchtigkeit der Erde im Topf.

Isoliert von der Erde in den Boden des Topfes 50 eingelassen befindet sich ein Schaltkreis 58. Dieser hat mehrere Funktionen: Einerseits ist er mit allen Sensoren verbunden, die am und im Topf 50 angebracht sind und verarbeitet, insbesondere digitalisiert, die von diesen Sensoren erhaltenen Parameterwerte; andererseits liefert er die digitalisierten Daten für die drahtlose Übermittlung an den zentralen Server SOS 6.

Diese drahtlose Übermittlung erfolgt über eine Antenne 59, die am oberen Rand des Topfes 50 angeordnet ist. Eine zweite Antenne 59' kann seitlich am Topf 50 angeordnet sein und ggf. wahlweise oder zusätzlich betrieben werden. Diese Antennen dienen in der Hauptsache zur Übermittlung der von den verschiedenen, im und am Topf angebrachten Sensoren ermittelten, digitalisierten Daten.

Wenn die (in den Fign. 5a und 5b nicht gezeigte) Pflanze im Topf 50 einen der im Zusammenhang mit den Fign. 4a und 4b beschriebenen Sensorclip 44 trägt oder ein solcher im Topf 50 angeordnet ist, kann eine der vorhandenen Antennen auch für den Empfang der vom Sensorclip 44 übermittelten Daten benutzt werden. Die Verarbeitung dieser Sensorclip-Daten erfolgt dann ebenfalls im Schaltkreis 58, der in diesem Fall als Relaisstation dient.

Die für den Betrieb des Schaltkreises 58 und damit der Übermittlung und ggf. der Sensoren erforderliche Energie liefert eine Batterie 60, die in einem von außen zugänglichen Fach des Topfes so untergebracht ist, dass sie gegenüber der Erde im Topf 50 isoliert ist und leicht ausgetauscht werden kann.

Fig. 5e zeigt eine Draufsicht eines wiederum leicht modifizierten smarten Blumentopfes. Hier sind zwei Lichtsensoren 52 und 52' auf dem oberen Rand des Topfes angebracht, dazu zwei RFID Tags 61 und 61' und zwei Nährstoffsensoren 55 und 55' an der Innenwand des Topfes. Am Boden desselben sind zwei Elektroden 56 für den Erdfeuchtigkeitssensor und ein Stickstoffsensor 57 angeordnet.

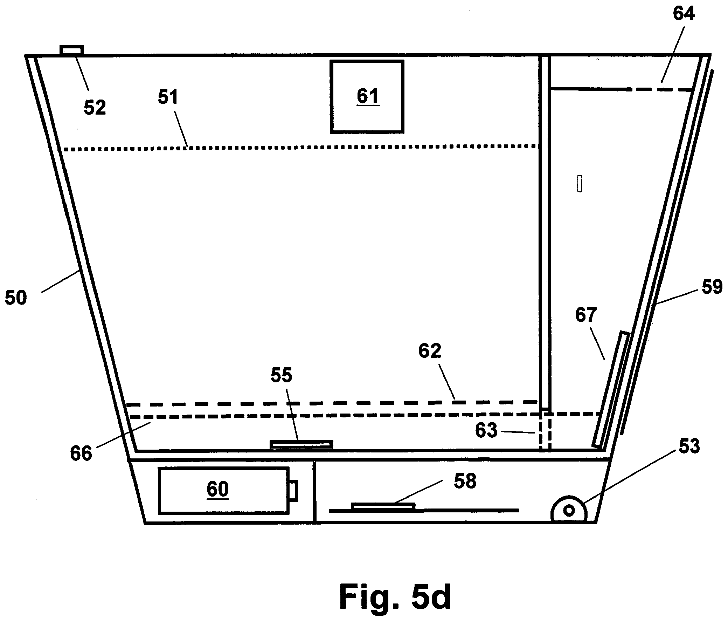

Die Fign. 5c und 5d zeigen eine weitere Ausführungen des smarten Blumentopfes, diesmal geeignet für Pflanze die per Unterflurbewässerung mit Wasser versorgt werden. Dabei wird der Wasserstandssensor 20 über einen elektronischen Schaltkreis an den smarten Blumentopf 50 derart angeschlossen, dass sie eine Einheit bilden. Dabei ist der Wasserstandssensor 20 über eine Verbindung 63 derart mit dem Innenraum des Topfes 50 verbunden, dass Wasser zum Wasserstandssensor 20 gelangen kann. Fig. 5d zeigt den intelligenten Blumentopf ohne den Wasserstandssensor 20 an. Das Problem der Wasserstandsmessung ist hier durch Elektroden 67 gelöst, die z.B. durch Widerstandsmessung oder per Kapazitätsmessung den Wasserstand messen können.

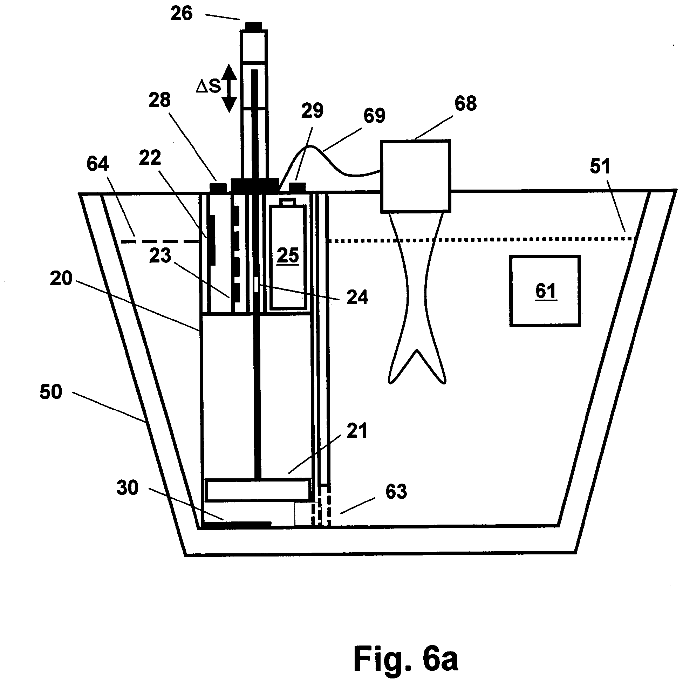

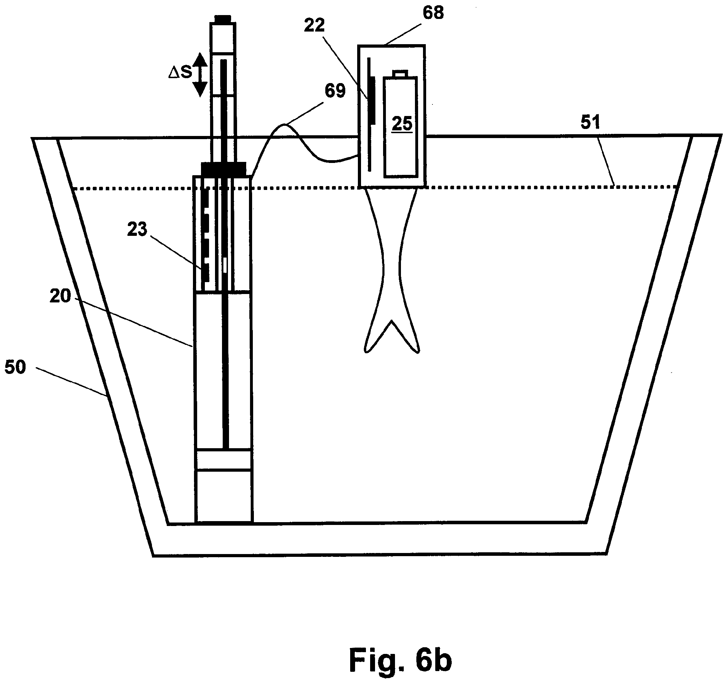

Zwei weitere Ausführungsbeispiele sind in den Fign. 6a und 6b gezeigt. In beiden werden der oben bereits genannte Wasserstandssensor 20 und ein Feuchtigkeitssensor 68 elektrisch zu einer Einheit kombiniert. Diese Lösung ist z. B. für eine Übergangszeit sinnvoll, wenn der Besitzer seine Pflanze nicht in den oben beschriebenen intelligenten Blumentopf umtopfen will, sondern sie im bisherigen Topfsystem belassen will. Dabei gibt es zwei Konfigurationsmöglichkeiten. Wie in Fig. 6a gezeigt, können sich der Schaltkreis 22, die Batterie 25, sowie das notwendige Funkmodul, das vorzugsweise Teil des Schaltkreises 22 ist, im Wasserstandssensor 20 befinden. Üblicherweise ergibt sich jedoch das Problem, dass insbesondere Batterien viel Platz verbrauchen und daher nur schwer in der Aussenwand des Wasserstandsanzeigers Platz finden.

In einer zweiten Konfiguration, die in Fig. 6b dargestellt ist, befinden sich der Schaltkreis 22, die Batterie 25, sowie das notwendige Funkmodul im Feuchtigkeitssensor 68. Bei diesem Sensor gibt es selten Platzprobleme, sodass eine grössere und damit kostengünstigere Batterie verwendet werden kann. Ausserdem befindet sich in dieser Konfiguration die Batterie im "Trockenbereich" ausserhalb des Topfes, was oft vorteilhaft sein kann. In beiden Konfigurationen werden der Wasserstands- und der Feuchtigkeitssensor über ein Kabel 69 miteinander verbunden. Dieses Kabel kann z.B. ein USB-Kabel sein. Dieses Kabel dient dazu, Daten zwischen den beiden Sensoren auszutauschen, sowie diese mit Energie zu versorgen. Daten fliessen über das Kabel von einem Sensor zum Schaltkreis 22 des anderen Sensors. Ebenso ist eine Stromversorgung durch die Batterie 25 auf diese Weise möglich.

Für den Fachmann wird es nun kein Problem darstellen, einen smarten Blumentopf zu entwerfen und herzustellen, der spezifische Bedingungen und Bedürfnisse erfüllt, ganz gleich ob von Seiten der Pflanze oder des Benutzers bzw. Besitzers.

## Patentansprüche

1. System zur Ermittlung des Zustands und/oder von Änderungen des Zustands- und/oder der Befindlichkeit einer Topfpflanze (1) und zur Anzeige des ermittelten Zustands und/oder der Änderung an den Besitzer/Benutzer mittels drahtloser Kommunikation, wobei
- auf oder in einem Nährmedium in einem Pflanzgefäss (2) oder einem Behälter (50) mehrere Sensoren oder Messfühler (3) angeordnet sind, die je mindestens einen physikalischen und/oder chemischen Parameter ermitteln, wobei jeder dieser Parameter als elektrisches Parametersignal dargestellt wird,
- im Pflanzgefäss (2) oder in einem der Sensoren (3, 23, 26, 28, 29, 30, 52, 55, 56, 67, 68) ein elektronischer Schaltkreis (22, 58) vorgesehen ist, der aus den ermittelten elektrischen Parametersignalen digital codierte Parametersignale erstellt und diese drahtlos vorzugsweise in Realzeit an einen ausserhalb des Pflanzgefässes (2) oder Behälters (50) befindlichen Empfänger (4) übermittelt,
**dadurch gekennzeichnet, dass**
- zumindest ein Sensor (3) zur Bestimmung des Nähr- bzw. Schadstoffgehalts des Nährmediums ausgebildet ist.

2. System nach Anspruch 1, **dadurch gekennzeichnet, dass**
- der zur Bestimmung des Nähr- bzw. Schadstoffgehalts des Nährmediums dienende Sensor (3) die Leitfähigkeit des im Behälter (50) befindlichen Nährmediums bzw. des im Behälter befindlichen Grundwassers ermittelt, vorzugsweise indem er mittels mindestens zweier Elektroden (32, 32') eine Widerstandsmessung durchführt.

3. System nach Anspruch 1 oder 2, **dadurch gekennzeichnet, dass**
- einer der Sensoren (3) für die Messung mindestens eines der folgenden physikalischen oder chemischen Parameter vorgesehen sind:
- Temperatur und/oder Feuchtigkeit des Nährmediums,
- Temperatur und/oder Feuchtigkeit der Umgebungsluft,
- Dauer und/oder Intensität und/oder Art der auf die Topfpflanze (1) fallenden Strahlung bzw. des Lichts.

4. System nach mindestens einem der vorhergehenden Ansprüche, **dadurch gekennzeichnet, dass**
- die digital codierten Parametersignale drahtlos über ein bestehendes Kommunikationsnetz, insbesondere ein W-LAN oder das Internet, übermittelt werden.

5. System nach mindestens einem der vorhergehenden Ansprüche, **dadurch gekennzeichnet, dass**
- mehrere Sensoren (55, 55', 55") zur Messung der Feuchtigkeit und/oder des Nähr- oder Schadstoffgehalts im Nährmedium vorgesehen sind, die in unterschiedlichen Höhen angeordnet sind, vorzugsweise am Boden und/oder an der Wand des Pflanzgefässes (2) oder Behälters (50).

6. System nach mindestens einem der vorhergehenden Ansprüche, **dadurch gekennzeichnet, dass**
- mehrere Sensoren (55, 55', 55") zur Messung der Feuchtigkeit und/oder des Nähr- oder Schadstoffgehalts im Nährmedium vorgesehen sind, wobei sich die Sensoren zur Messung der Feuchtigkeit vom Boden des Pflanzgefässes (2) oder Behälters (50) unterschiedlich weit in das Nährmedium hinein erstrecken und die Sensoren zur Messung des Nähr- oder Schadstoffgehalts in unterschiedlicher Höhe des Pflanzgefässes (2) oder Behälters (50) angeordnet sind.

7. System nach mindestens einem der vorhergehenden Ansprüche, **dadurch gekennzeichnet, dass**
- zumindest ein Sensor (3) zur Bestimmung der Feuchtigkeit im Nährmedium ausgebildet ist, wobei die Messung durch Feststellung eines FlüssigkeitsNiveaus mittels eines mit einem Magneten verbundenen Schwimmer-Messgeräts (21) erfolgt.

8. System nach mindestens einem der vorhergehenden Ansprüche, **dadurch gekennzeichnet, dass**
- ein RFID-Tag (61) am Pflanzgefäss (2) oder Behälter (50) angbringbar ist, der die Identifikation des Behälters (50) mittels eines RFID-Lesegeräts ermöglicht.

9. System nach mindestens einem der vorhergehenden Ansprüche, **dadurch gekennzeichnet, dass**
- ein Sensor als mobiler Sensorclip (33) ausgebildet ist, der an oder in unmittelbarer Nähe der Topfpflanze (1) anbringbar ist und der eine Stromversorgung (35), mehrere Sensoren (34) und einen Schaltkreis (36) zur Verarbeitung und vorzugsweise drahtlosen Übermittlung der von den Sensoren ermittelten elektrischen Parametersignale an den elektronischen Schaltkreis des zugeordneten Pflanzgefässes (2) aufweist.

10. System nach Anspruch 9, **dadurch gekennzeichnet, dass**
- der mobile Sensorclip (33) näherungsweise die Form eines Blattes, einer Frucht oder einer Blüte aufweist, wobei die Oberfläche oder Teile davon (35) als Solarzelle für die Stromversorgung ausgebildet ist.

11. System nach mindestens einem der vorhergehenden Ansprüche, **dadurch gekennzeichnet, dass**
- zumindest einer der Sensoren (20 bis 26) unmittelbar digital codierte Parametersignale erstellt.

12. System nach Anspruch 11, **dadurch gekennzeichnet, dass**
- mehr als zwei Sensoren (3) vorgesehen sind, von denen zumindest zwei, vorzugsweise zwei nahe beieinander angeordnete, mittels einer Leitung verbunden sind, während die anderen Sensoren drahtlos vernetzt sind.

13. System nach mindestens einem der vorhergehenden Ansprüche, **dadurch gekennzeichnet, dass**
- zumindest ein Sensor (52) zur Messung des einfallenden Lichts, insbesondere zur Ermittlung von dessen Dauer und/oder Intensität und/oder Art, am oberen Ende oder auf der oberen Kante des Behälters (50) oder Pflanzgefässes, vorzugsweise mehrere Sensoren (52, 52') an mehreren Stellen angeordnet ist/sind.

14. Verfahren zum Betrieb eines Systems nach einem der Ansprüche 1 bis 13 zur Ermittlung von Zustand, Zustands- und/oder Befindlichkeitsänderungen einer Pflanze (1) und zur Anzeige des ermittelten Zustands an dessen Besitzer bzw. Benutzer mittels drahtloser Kommunikation,
**dadurch gekennzeichnet, dass**
- auf, an oder in einem Pflanzgefäss (2) oder Behälter (50) von mehreren Sensoren oder Messfühlern (23, 26, 28, 29, 30) mindestens zwei physikalische und/oder chemische Parameter vorzugsweise in Realzeit ermittelt werden, wovon ein Parameter den Nähr- bzw. Schadstoffgehalts des Nährmediums anzeigt,
- aus den ermittelten Parametern digital codierte Parametersignale erstellt werden, die vorzugsweise drahtlos an einen Empfänger (4) übermittelt werden,
- in einem Empfänger (4) die empfangenen Parametersignale verarbeitet und daraus Ausgangssignale erzeugt werden, die eine Aussage über den Zustand des Objekts (1) darstellen und dem Besitzer bzw. Benutzer vorzugsweise drahtlos an ein mobiles Anzeigegerät (11) übermittelt werden und
- dieses Anzeigegerät (11) dem Besitzer bzw. Benutzer vorzugsweise in Realzeit eine optische und/oder akustische Anzeige über den Zustand der Pflanze (2) vermittelt.

15. Verfahren nach Anspruch 14, **dadurch gekennzeichnet, dass**
- der Benutzer, vorzugsweise in Realzeit, alarmiert wird, wenn das digital codierte Parametersignal einer überwachten Pflanze (2) oder die daraus abgeleiteten Ausgangsignale einen für die Pflanze bedrohlichen Zustand darstellen oder absehbar in naher Zukunft einen solchen Zustand darstellen können.

## Claims

1. System for detecting the state and/or changes in the state and/or condition of a potted plant (1) and for indicating the detected state and/or the change to the owner/user by means of wireless communication,
- a plurality of sensors or transducers (3) being arranged on or in a nutrient medium in a planter (2) or a container (50), each sensor or transducer detecting at least one physical and/or chemical parameter and each of said parameters being represented as an electric parameter signal,
- an electronic circuit (22, 58) being provided in the planter (2) or in one of the sensors (3, 23, 26, 28, 29, 30, 52, 55, 56, 67, 68) which circuit creates digitally coded parameter signals from the detected electric parameter signals and wirelessly transmits said digitally coded parameter signals, preferably in real time, to a receiver (4) located outside the planter (2) or container (50),
**characterised in that**
- at least one sensor (3) is designed to determine the nutrient or pollutant content of the nutrient medium.

2. System according to claim 1, **characterised in that**
- the sensor (3) used for determining the nutrient or pollutant content of the nutrient medium detects the conductivity of the nutrient medium located in the container (50) or of the groundwater located in the container, preferably by said sensor carrying out a resistance measurement by means of at least two electrodes (32, 32').

3. System according to either claim 1 or claim 2, **characterised in that**
- one of the sensors (3) is provided for measuring at least one of the following physical or chemical parameters:
- temperature and/or moisture of the nutrient medium,
- temperature and/or humidity of the ambient air,
- duration and/or intensity and/or type of radiation incident on the potted plant (1) or of light.

4. System according to at least one of the preceding claims, **characterised in that**
- the digitally coded parameter signals are transmitted via an existing communication network, in particular a WLAN or the Internet.

5. System according to at least one of the preceding claims, **characterised in that**
- a plurality of sensors (55, 55', 55") are provided for measuring the moisture and/or the nutrient or pollutant content in the nutrient medium, which sensors are arranged at various heights, preferably on the bottom and/or the wall of the planter (2) or container (50).

6. System according to at least one of the preceding claims, **characterised in that**
- a plurality of sensors (55, 55' 55") are provided for measuring the moisture and/or the nutrient or pollutant content in the nutrient medium, the sensors for measuring the moisture extending from the bottom of the planter (2) or container (50) into the nutrient medium to various extents and the sensors for measuring the nutrient or pollutant content being arranged at various heights of the planter (2) or container (50).

7. System according to at least one of the preceding claims, **characterised in that**
- at least one sensor (3) is designed to determine the moisture in the nutrient medium, the measurement taking place by ascertaining a liquid level by means of a liquid level float (21) connected to a magnet.

8. System according to at least one of the preceding claims, **characterised in that**
- an RFID tag (61) can be attached to the planter (2) or container (50), which tag makes it possible to identify the container (50) by means of an RFID reader.

9. System according to at least one of the preceding claims, **characterised in that**
- one sensor is designed as a mobile sensor clip (33) which can be attached to or in the immediate vicinity of the potted plant (1) and which has a power supply unit (35), a plurality of sensors (34) and a circuit (36) for processing and preferably wirelessly transmitting the electric parameter signals detected by the sensors to the electronic circuit of the associated planter (2).

10. System according to claim 9, **characterised in that**
- the mobile sensor clip (33) has approximately the shape of a leaf, a fruit or a blossom, the surface or parts thereof (35) being designed as a solar cell for the power supply.

11. System according to at least one of the preceding claims, **characterised in that** at least one of the sensors (20 to 26) creates immediately digitally coded parameter signals.

12. System according to claim 11, **characterised in that**
- more than two sensors (3) are provided, of which at least two, preferably two arranged close together, are connected by means of a line, while the other sensors are networked wirelessly.

13. System according to at least one of the preceding claims, **characterised in that**
- at least one sensor (52) for measuring the incident light, in particular for detecting the duration and/or intensity and/or type thereof, is arranged at the top end or on the top edge of the container (50) or planter, preferably a plurality of sensors (52, 52') are arranged at a plurality of points.

14. Method for operating a system according to any of claims 1 to 13 for detecting the state and/or changes in the state and/or condition of a plant (1) and for indicating the detected state to the owner or user of said plant by means of wireless communication,
**characterised in that**
- on or in a planter (2) or container (50), at least two physical and/or chemical parameters are detected, preferably in real time, by a plurality of sensors or transducers (23, 26, 28, 29, 30) of which one parameter indicates the nutrient or pollutant content of the nutrient medium,
- digitally coded parameter signals are created from the detected parameters and transmitted, preferably wirelessly, to a receiver (4),
- in a receiver (4), the received parameter signals are processed and output signals are produced therefrom which provide information regarding the state of the object (1) and are transmitted, preferably wirelessly, to the owner or user on a mobile indicator (11), and
- said indicator (11) conveys to the owner or user, preferably in real time, an optical and/or acoustic indication of the state of the plant (2).

15. Method according to claim 14, **characterised in that**
- the user is alerted, preferably in real time, if the digitally coded parameter signal of a monitored plant (2) or the output signals derived therefrom represent a state in which the plant is threatened or can foreseeably represent such a state in the near future.

## Revendications

1. Système de détermination de l'état et/ou de variations de l'état et/ou des conditions d'une plante en pot (1), et d'indication de l'état déterminé et/ou de la variation déterminée au propriétaire/à l'utilisateur au moyen d'une communication sans fil, dans lequel
- plusieurs capteurs ou sondes de mesure (3) sont disposés sur ou dans un milieu nutritif dans un bac à plantes (2) ou un récipient (50), et déterminent chacun au moins un paramètre physique et/ou chimique, dans lequel chacun desdits paramètres est représenté sous la forme d'un signal de paramètre électrique,
- un circuit électronique (22, 58) est prévu dans le bac à plantes (2) ou dans l'un des capteurs (3, 23, 26, 28, 29, 30, 52, 55, 56, 67, 68) et génère à partir des signaux de paramètres électriques déterminés des signaux de paramètres codés numériquement et les transmet sans fil, de préférence en temps réel, à un récepteur (4) se trouvant à l'extérieur du bac à plantes (2) ou du récipient (50),
**caractérisé en ce que**
- au moins un capteur (3) est conçu pour déterminer la teneur en nutriments ou en polluants du milieu nutritif.

2. Système selon la revendication 1, **caractérisé en ce que**
- le capteur (3) servant à déterminer la teneur en nutriments ou en polluants du milieu nutritif détermine la conductivité du milieu nutritif se trouvant dans le récipient (50) ou de l'eau de sol se trouvant dans le récipient, de préférence en effectuant une mesure de résistance au moyen d'au moins deux électrodes (32, 32').

3. Système selon la revendication 1 ou 2, **caractérisé en ce que**
- l'un des capteurs (3) est prévu pour mesurer au moins l'un des paramètres physiques ou chimiques suivants :
- la température et/ou l'humidité du milieu nutritif,
- la température et/ou l'humidité de l'air ambiant,
- la durée et/ou l'intensité et/ou le type de rayonnement ou de lumière incident sur la plante en pot (1) .

4. Système selon au moins l'une des revendications précédentes, **caractérisé en ce que**
- les signaux de paramètres codés numériquement sont transmis sans fil par l'intermédiaire d'un réseau de communication existant, en particulier un W-LAN ou l'Internet.

5. Système selon au moins l'une des revendications précédentes, **caractérisé en ce que**
- plusieurs capteurs (55, 55', 55") sont prévus pour mesurer l'humidité et/ou la teneur en éléments nutritifs ou en polluants du milieu nutritif, et sont disposés à différentes hauteurs, de préférence au fond et/ou sur la paroi du bac à plantes (2) ou du récipient (50) .

6. Système selon au moins l'une des revendications précédentes, **caractérisé en ce que**
- plusieurs capteurs (55, 55', 55") sont prévus pour mesurer l'humidité et/ou la teneur en nutriments ou en polluants du milieu nutritif, dans lequel les capteurs destinés à mesurer l'humidité s'étendent depuis le fond du bac à plantes (2) ou du récipient (50) à différentes distances dans le milieu nutritif et les capteurs destinés à mesurer la teneur en nutriments ou en polluants sont disposés à différentes hauteurs du bac à plantes (2) ou du récipient (50).

7. Système selon au moins l'une des revendications précédentes, **caractérisé en ce que**
- au moins un capteur (3) est conçu pour déterminer l'humidité dans le milieu nutritif, dans lequel la mesure est effectuée en déterminant un niveau de liquide au moyen d'un dispositif de mesure à flotteur (21) relié à un aimant.

8. Système selon au moins l'une des revendications précédentes, **caractérisé en ce que**
- une étiquette RFID (61) peut être fixée au bac à plantes (2) ou au récipient (50), et permet l'identification du récipient (50) au moyen d'un lecteur RFID.

9. Système selon au moins l'une des revendications précédentes, **caractérisé en ce que**
- un capteur est réalisé sous la forme d'un capteur mobile à pince (33) qui peut être fixé à la plante en pot (1) ou à proximité immédiate de celle-ci et qui comporte une alimentation électrique (35), plusieurs capteurs (34) et un circuit (36) destiné à traiter et à transmettre, de préférence sans fil, les signaux de paramètres électriques déterminés par les capteurs au circuit électronique du bac à plantes (2) associé.

10. Système selon la revendication 9, **caractérisé en ce que**
- le capteur mobile à pince (33) présente approximativement la forme d'une feuille, d'un fruit ou d'une fleur, sa surface ou des parties de celle-ci (35) étant réalisées sous la forme d'une cellule solaire pour l'alimentation électrique.

11. Système selon au moins l'une des revendications précédentes, **caractérisé en ce que**
- au moins l'un des capteurs (20 à 26) génère directement des signaux de paramètres codés numériquement.

12. Système selon la revendication 11, **caractérisé en ce que**
- plus de deux capteurs (3) sont prévus, dont au moins deux, de préférence deux capteurs disposés à proximité l'un de l'autre, sont reliés au moyen d'une ligne, alors que les autres capteurs sont connectés en réseau sans fil.

13. Système selon au moins l'une des revendications précédentes, **caractérisé en ce que**
- au moins un capteur (52) destiné à mesurer la lumière incidente, en particulier à déterminer sa durée et/ou son intensité et/ou son type, est disposé à l'extrémité supérieure ou sur le bord supérieur du récipient (50) ou du bac à plantes, de préférence plusieurs capteurs (52, 52') en plusieurs emplacements.

14. Procédé pour faire fonctionner un système selon l'une des revendications 1 à 13 afin de déterminer l'état, des variations de l'état et/ou des conditions d'une plante (1) et d'indiquer l'état déterminé à son propriétaire ou à son utilisateur au moyen d'une communication sans fil, **caractérisé en ce que**
- au moins deux paramètres physiques et/ou chimiques sont déterminés, de préférence en temps réel, sur, au niveau ou à l'intérieur d'un bac à plantes (2) ou d'un récipient (50) par plusieurs capteurs ou sondes de mesure (23, 26, 28, 29, 30), parmi lesquels un paramètre indique la teneur en nutriments ou en polluants du milieu nutritif,
- des signaux de paramètres codés numériquement sont générés à partir des paramètres déterminés et sont transmis, de préférence sans fil, à un récepteur (4),
- les signaux de paramètres reçus sont traités dans un récepteur (4) et des signaux de sortie qui représentent une indication de l'état de l'objet (1) sont générés à partir de ceux-ci et sont transmis au propriétaire ou à l'utilisateur, de préférence sans fil, sur un appareil indicateur mobile (11), et
- ledit appareil indicateur (11) fournit au propriétaire ou à l'utilisateur, de préférence en temps réel, une indication optique et/ou acoustique de l'état de la plante (2).

15. Procédé selon la revendication 14, **caractérisé en ce que**
- l'utilisateur est alerté, de préférence en temps réel, lorsque le signal de paramètre codé numériquement d'une plante surveillée (2) ou les signaux de sortie qui en sont dérivés représentent un état menaçant pour la plante ou peuvent probablement représenter un tel état dans un avenir proche.
